# EUROPEAN PATENT APPLICATION

(11) **EP 3 970 510 A1**
(43) Date of publication of application: **23.03.2022**
(21) Application number: 19928555.2
(22) Date of filing: 13.05.2019
(51) Int. Cl.: A23L 29/30, A23K 20/163, A23L 33/125, A61K 8/73, A61K 47/36, C05G 3/00, C12N 1/00, A23D 7/005, A23D 9/007, A23G 1/40, A23G 3/00, A23G 3/34, A23L 7/126, A23L 9/20, A23L 11/00, A23L 21/10, A23L 27/50, A23L 27/60, C08B 30/18, C12P 19/16, C13B 40/00

(54) **MODIFIER, MODIFICATION COMPOSITION COMPRISING SAID MODIFIER, FOOD AND BEVERAGE, DRUG, COSMETIC, INDUSTRIAL PRODUCT, FEED, MEDIUM OR FERTILIZER USING SAME, AND METHOD FOR MODIFYING SAID PRODUCTS**

(71) Applicant: Showa Sangyo Co., Ltd., Tokyo 101-8521 (JP)
(72) Inventor: KAWANO Atsushi, Tokyo 101-8521 (JP); YAMAMOTO Tomohiro, Tokyo 101-8521 (JP)
(74) Representative: Forrest, Stuart
(86) International application number: PCT/JP2019/018952
(87) International publication number: WO 2020/230238

(57) **Abstract**

Provided is a novel application of a starch decomposition product. The present technology provides a modifier that includes a starch decomposition product in which the content with a glucose polymerization degree (DP) of 8 to 19 is 32% or more, and the content with a glucose polymerization degree (DP) of 20 or more is 30% or less, and is configured to partially or completely crystallize the starch decomposition product together with raw materials of a target product, thereby modifying qualities of the target product. The modifier used in the present technology can modify qualities of a target product, such as humidity resistance, solidifying properties, gelling properties, shape retainability, plasticity, whiteness, water separation resistance, oil separation resistance, mouthfeel, tactile sensation, aftertaste, creaming properties, heat resistance, thickening properties, and aging resistance.

## Description

### Technical Field

The present technology relates to a modifier for modifying qualities of a target product such as a food or beverage, a pharmaceutical product, a cosmetic product, an industrial product, a feed, a medium, or a fertilizer. It more specifically relates to a modifier for modifying qualities of a target product such as a food or beverage, a pharmaceutical product, a cosmetic product, an industrial product, a feed, a medium, or a fertilizer; a composition for modification containing the modifier; a food or beverage, a pharmaceutical product, a cosmetic product, an industrial product, a feed, a medium, or a fertilizer using the same; and a method for modifying these products.

### Background Art

Conventionally, in the field of foods and beverages, starch decomposition products have been utilized for applications such as sweeteners, taste quality adjustment, osmotic pressure adjustment, moisturizers, and powdered substrates. In addition, also in the field of pharmaceutical products, starch decomposition products have been utilized for applications such as carbohydrate sources for enteral nutrients and excipients for medicines. Further, in the field of cosmetic products, starch decomposition products have also been utilized for applications such as binders for solidifying cosmetic products and viscosity adjustment for creamy cosmetic products.

Like this, a starch decomposition product is utilized for various applications as described above by adjusting its basic physical properties such as the degree of sweetness, taste quality, osmotic pressure, viscosity, and moisture absorbency. For example, those having a high degree of sweetness are suitable for use as sweeteners, and, conversely, those having a low degree of sweetness are suitable for taste quality adjusters, osmotic pressure adjusters, powdered substrates, and the like. In addition, the moisture absorbency of the starch decomposition product itself, for example, also serves as an important factor in selecting its application. For example, when the moisture absorbency is too high, such a starch decomposition product may solidify during storage or distribution or become sticky, and thus is not suitable for utilization in a powdered food or application to a powdered substrate or the like.

In addition, starch decomposition products obtained by crystallizing these starch decomposition products have also been utilized in various fields taking advantage of their characteristics, such as moisture absorbency. For example, Patent Literature 1 discloses a technology for producing amylose particles that can be used in the field of foods, the field of pharmaceutical products, the field of cosmetic products, and the like, according to which cyclomaltodextrin-glucanotransferase is allowed to act on a cyclodextrin- or starch-containing aqueous solution to generate insoluble amylose particles in the aqueous solution, and the amylose particles are collected.

In addition, Patent Literature 2 discloses a technology for producing microspherical crystallites that can be used as additives for cosmetic products, carriers for active substances in pharmaceutical and other applications, food additives, fillers for biodegradable polymers or industrial polymers, and the like, according to which 1,4-α-D-polyglucan or a polysaccharide is melted in water, the melt is led to precipitate, the resulting mixture is cooled, and the formed particles are separated.

### Citation List

### Patent Literature

Patent Literature 1: JP-A-04-85301
Patent Literature 2: Published Japanese Translation of PCT Patent Application No. 2004-512405

### Summary of Invention

### Technical Problem

A main object of the present technology is to provide a novel application of a starch decomposition product.

### Solution to Problem

The inventors of the present application have conducted extensive research on the applications of existing starch decomposition products. As a result, the inventors of the present application have found out that when a starch decomposition product characterized by having high contents of, among oligosaccharides, a component having an extremely high molecular weight and a low-molecular-weight dextrin component is crystallized together with raw materials of a target product, qualities of the target product are modified, and thus accomplished the present technology.

That is, the present technology provides, first, a modifier including a starch decomposition product in which
the content with a glucose polymerization degree (DP) of 8 to 19 is 32% or more, and
the content with a glucose polymerization degree (DP) of 20 or more is 30% or less,
the modifier being configured to partially or completely crystallize the starch decomposition product together with raw materials of a target product, thereby modifying qualities of the target product.

It is possible that the starch decomposition product used in the modifier according to the present technology has an iodine coloration value of 0.15 or more.

It is possible that the starch decomposition product used in the modifier according to the present technology is obtained by allowing at least a debranching enzyme and a branching enzyme to act on a starch or a starch decomposition intermediate.

In addition, it is possible that the starch decomposition product used in the modifier according to the present technology is obtained by allowing at least a debranching enzyme to act on a starch or a starch decomposition intermediate that has been liquefied with an acid.

As target products that can be modified by the modifier according to the present technology, foods and beverages, pharmaceutical products, cosmetic products, industrial products, feeds, media, and fertilizers can be mentioned.

The modifier according to the present technology can also be distributed as a composition for modification.

The modifier and the composition for modification according to the present technology can be used for foods and beverages, pharmaceutical products, cosmetic products, industrial products, feeds, media, and fertilizers.

That is, the present technology provides a food or beverage, a pharmaceutical product, a cosmetic product, an industrial product, a feed, a medium, or a fertilizer including a starch decomposition product in which
the content with a glucose polymerization degree (DP) of 8 to 19 is 32% or more, and
the content with a glucose polymerization degree (DP) of 20 or more is 30% or less, and which has been partially or completely crystallized together with raw materials of the food or beverage, pharmaceutical product, cosmetic product, industrial product, feed, medium, or fertilizer.

The present technology next provides a method for modifying a target product, including a crystallization step of partially or completely crystallizing, together with raw materials of the target product, a starch decomposition product in which
the content with a glucose polymerization degree (DP) of 8 to 19 is 32% or more, and
the content with a glucose polymerization degree (DP) of 20 or more is 30% or less.

Here, the technical terms used in the present technology will be described. "Debranching enzyme" is a general term for enzymes that catalyze the reaction of hydrolyzing α-1,6-glucoside bonds, which are branch points in starch. For example, "Isoamylase (glycogen 6-glucanohydrolase)", "Pullulanase (pullulan 6-glucan hydrolase)", and "amylo-1,6-glucosidase/4-α-glucanotransferase" are known. Incidentally, these debranching enzymes may be used in combination according to the purpose.

"Branching enzyme" is a general term for enzymes that function to act on a linear glucan linked by α-1,4-glucoside bonds to form α-1,6-glucoside bonds. They are present in animals, bacteria, and the like, and can also be purified from plants such as potatoes, rice seeds, and corn seeds.

### Advantageous Effects of Invention

The starch decomposition product used in the modifier according to the present technology contains large amounts of a high-molecular-weight oligosaccharide component and a low-molecular-weight dextrin component (glucose polymerization degree: DP 8 to 19). Accordingly, an interaction between linear sugar molecules, which does not occur in low-molecular-weight oligosaccharides, occurs, resulting in high crystallinity. By utilizing this nature, qualities of various target products can be modified.

### Brief Description of Drawings

Fig. 1 is a drawing-substituting photograph, showing micrographs (×400) 0 minutes, 10 minutes, 30 minutes, and 180 minutes after mixing the ingredients of the fat spread of Sample 13 of Experiment Example 5 and the starch decomposition product of Example 3.
Fig. 2 is a drawing-substituting photograph, showing a micrograph (×400) with the crystallized starch decomposition product of Example 3 being added 180 minutes after mixing the ingredients of the fat spread of Sample 13 of Experiment Example 5.
Fig. 3 is a drawing-substituting photograph, showing Samples 1, 3, and 4 of Experiment Example 2.
Fig. 4 is a drawing-substituting photograph, showing Samples 5 and 6 of Experiment Example 3.
Fig. 5 is a drawing-substituting photograph, showing Samples 9 and 11 of Experiment Example 4 after being stored at 4°C for 24 hours.
Fig. 6 is a drawing-substituting photograph, showing Samples 13 and 16 of Experiment Example 5.
Fig. 7 is a drawing-substituting photograph, showing Samples 17 and 21 of Experiment Example 6.

### Description of Embodiments

Hereinafter, preferred modes for carrying out the present technology will be described. Incidentally, the embodiments described below show some examples of typical embodiments of the present technology, and do not narrow the interpretation of the scope of the present technology.

### <About Starch Decomposition Product>

The starch decomposition product used in the present technology is obtainable by decomposing (saccharifying) a starch raw material, examples thereof including starches such as corn starch, waxy corn starch, rice starch, and wheat starch (starches of on-ground type), underground stem- or root-derived starches such as potato starch, tapioca starch, and sweet potato starch (starches of underground type), and processed starches thereof. The starch raw material used is not particularly limited, and any starch raw material can be used.

As a compositional characteristic of the starch decomposition product used in the present technology, the content with a glucose polymerization degree (hereinafter referred to as "DP") of 8 to 19 is 32% or more, and the content with a DP of 20 or more is 30% or less. The starch decomposition product used in the present technology contains large amounts of a high-molecular-weight oligosaccharide component and a low-molecular-weight dextrin component (DP 8 to 19), and thus exhibits lower sweetness, lower osmotic pressure, and higher humidity resistance compared to general oligosaccharides. In addition, because the content with a DP of 20 or more is low, the flavor peculiar to dextrin, which may impair the flavor of the food or beverage, etc., is reduced. Accordingly, suitable application to applications where sweetness is not required is possible. For example, it can be used for food additives, foods and beverages, and medicines, in which use of an oligosaccharide having a high degree of sweetness is not desirable. In addition, even for foods and beverages, etc., in which use of dextrin has been difficult due to the strong flavor peculiar to dextrin, it can be used without impairing the flavor of the food or beverage, etc. The starch decomposition product used in the present technology is characterized in that because large amounts of a high-molecular-weight oligosaccharide component and a low-molecular-weight dextrin component (DP 8 to 19) are contained, an interaction between linear sugar molecules, which does not occur in low-molecular-weight oligosaccharides, occurs, leading to high crystallization ability.

Further, whereas an interaction between linear sugar molecules, which does not occur in low-molecular-weight oligosaccharides, occurs because of the presence of large amounts of a high-molecular-weight oligosaccharide component and a low-molecular-weight dextrin component (DP 8 to 19), because the content with a DP of 20 or more is low, that is, because the number of linear sugar molecules with a DP of 20 or more is also small, the starch decomposition product used in the present technology has moderate crystallization ability. Accordingly, excessive crystallization during production or use is suppressed, and thus it can be used for applications where crystallization is utilized.

Further, the starch decomposition product used in the present technology can be used in such a way that a non-crystallized starch decomposition product dissolved in water or soluble in water is efficiently produced, or a starch decomposition product is uniformly mixed with raw materials of a target product prior to crystallization and then crystallized.

In the starch decomposition product used in the present technology, the content with a DP of 8 to 19 is not particularly limited as long as it is 32% or more, but is preferably 40% or more, and more preferably 50% or more. This is because as the content with a DP of 8 to 19 increases, the crystallinity is enhanced, and even lower viscosity, lower sweetness, lower osmotic pressure, and higher humidity resistance are exhibited.

In addition, in the starch decomposition product used in the present technology, the content with a DP of 20 or more is not particularly limited as long as it is 30% or less, but is preferably 28% or less, more preferably 26% or less, and still more preferably 25% or less. This is because as the content with a DP of 20 or more decreases, the flavor peculiar to dextrin is more reduced.

The starch decomposition product used in the present technology preferably has an iodine coloration value of 0.15 or more. In the present technology, the iodine coloration value of a starch decomposition product is a value measured by the following iodine coloration value measurement method.

(Iodine Coloration Value Measurement Method) In a test tube having dispensed therein 5 ml of water, 25 mg (as solids) of a sample (starch decomposition product) was added and mixed, and then 100 µl of an iodine coloring solution (0.2 mass/vol% iodine and 2 mass/vol% potassium iodide) was added and stirred. After the mixture was allowed to stand at 30°C for 20 minutes, the absorbance at 660 nm was measured with a spectrophotometer using a glass cell having an optical path length of 10 mm, and the difference from the absorbance measurement value in the case of adding no sample was defined as the iodine coloration value.

The coloration reaction with iodine indicates the presence of linear sugar chains with a DP of 16 or more. In a starch decomposition product having a high content with a DP of 20 or more, a large number of linear sugar chains with a DP of 16 or more are present, and thus a coloration reaction is exhibited. Meanwhile, in a starch decomposition product having a low content with a DP of 20 or more, a coloration reaction is usually not exhibited, or even if exhibited, the iodine coloration value is extremely low. However, in the starch decomposition product used in the present technology, although the content with a DP of 20 or more is low, because the DP of the maim component is 8 to 19, which is near the lower limit of iodine coloration, and also because the amount of linear component is large, a coloration reaction with iodine is exhibited. That is, in a starch decomposition product in which the content with a DP of 20 or more is low, the iodine coloration value serves as an index indicating the degree of the linear component content.

In the present technology, as a result of using a starch decomposition product having an iodine coloration value of 0.15 or more, an interaction between linear sugar molecules, which does not occur in low-molecular-weight oligosaccharides, is even more likely to occur, making it possible to provide a modifier having even higher crystallization ability.

### <About Method for Producing Starch Decomposition Product>

The method for acquiring the starch decomposition product used in the present technology is not particularly limited as long as the advantages of the present technology are not impaired. For example, a starch decomposition product can be obtained by subjecting a starch raw material to a general treatment with an acid or enzyme or an appropriate combination of predetermined operations such as various chromatographies, membrane separation, and ethanol precipitation.

One method for efficiently obtaining the starch decomposition product used in the present technology is a method in which at least a debranching enzyme and a branching enzyme are allowed to act on a starch or a starch decomposition intermediate. A debranching enzyme is an enzyme that is involved in the decomposition of branched chains of starch, while a branching enzyme is an enzyme that is used for the synthesis of branched chains of starch. Therefore, the two are usually not used together. However, as a result of combined use of the two enzymes that show completely opposite actions, the starch decomposition product used in the present technology can be reliably produced. In this case, the order of action of the two enzymes is that the debranching enzyme is allowed to act simultaneously with or after the action of the branching enzyme.

The debranching enzyme is not particularly limited. For example, Pullulanase (pullulan 6-glucan hydrolase) and amylo-1,6-glucosidase/4-α-glucanotransferase can be mentioned, and, as a more preferred example, Isoamylase (glycogen 6-glucanohydrolase) can be used.

In addition, the branching enzyme is not particularly limited either. For example, those purified from animals, bacteria, and the like, those purified from plants such as potatoes, rice seeds, and corn seeds, commercially available enzyme preparations, and the like can be used.

As another example of the production method, there also is a method in which an acid is added to a starch or a starch decomposition intermediate to liquefy the same, and then a debranching enzyme is allowed to act thereon. As the acid that can be used at this time, one or more kinds of general acids can be freely selected and used as long as they are acids that can liquefy a starch or a starch decomposition intermediate and do not impair the advantages of the present technology. For example, hydrochloric acid, oxalic acid, sulfuric acid, and the like can be suitably applied.

In the method for producing the starch decomposition product used in the present technology, it is also possible to perform a step of removing impurities after the enzyme reaction. The method for removing impurities is not particularly limited, and one or more kinds of known methods can be freely combined and used. For example, methods such as filtration, activated carbon decolorization, and ion-exchage purification can be mentioned.

Further, although the starch decomposition product used in the present technology can be used as a liquid product containing the starch decomposition product that has undergone the enzyme reaction, it can also be dried by vacuum drying, spray drying, freeze drying, or the like and powdered. It is also possible that some components are fractionated by chromatography or membrane separation and used.

### <About Modifier and Composition for Modification Containing Starch Decomposition Product>

The modifier and the composition for modification according to the present technology each include the starch decomposition product described above, and are each configured to partially or completely crystallize the starch decomposition product together with raw materials of a target product, thereby modifying qualities of the target product. The starch decomposition product used in the present technology is, for example, crystallized together with raw materials of a target product such as a food or beverage, a pharmaceutical product, a cosmetic product, an industrial product, a feed, a medium, or a fertilizer, whereby qualities of these target products, such as humidity resistance, solidifying properties, gelling properties, shape retainability, plasticity, whiteness, water separation resistance, oil separation resistance, mouthfeel, tactile sensation, aftertaste, creaming properties, heat resistance, thickening properties, and aging resistance, can be modified.

The mechanism of modification by the modifier and the composition for modification according to the present technology is not clear, but is presumably such that the starch decomposition product forms fine crystals with the elapse of time and fills up the gaps in the target product, causing changes in the physical properties of the target product.

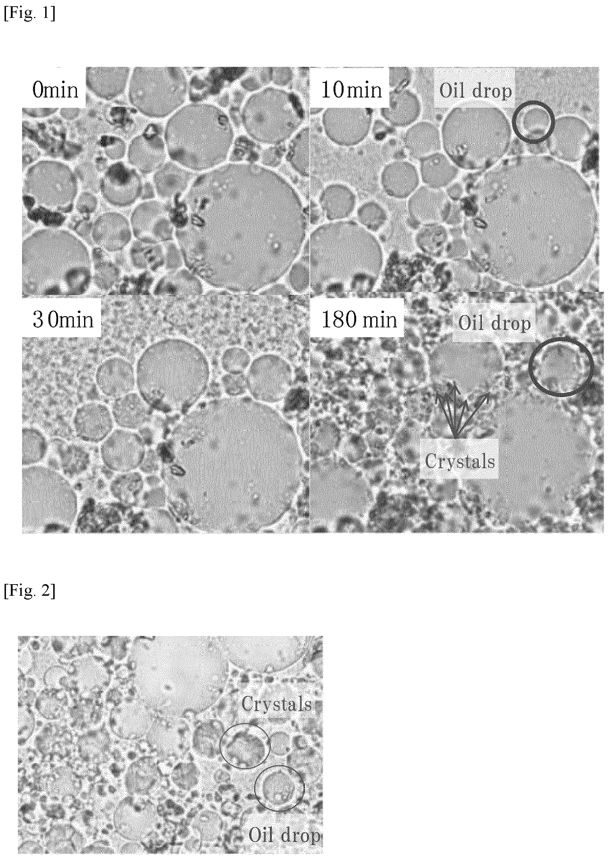

As an example, Fig. 1 shows micrographs (×400) 0 minutes, 10 minutes, 30 minutes, and 180 minutes after mixing the ingredients of the fat spread of Sample 13 of Experiment Example 5 described below. As shown in Fig. 1, it was confirmed that the starch decomposition product formed fine crystals with the elapse of time and filled up the gaps between oil droplets. The produced fat spread solidified like a cream, had shape retainability, was smooth and easy to spread on bread, and exhibited smooth mouthfeel.

Meanwhile, Fig. 2 shows a micrograph (×400) with a crystallized starch decomposition product being added 180 minutes after mixing the ingredients of the fat spread of Sample 13 of Experiment Example 5. As shown in Fig. 2, it was confirmed that when an already crystallized starch decomposition product was added after the production of the fat spread, the crystals were larger than in Fig. 1. The fat spread produced was not creamed and had rough mouthfeel.

As shown in these micrographs, presumably, in the modifier and the composition for modification according to the present technology, crystallization occurs together with raw materials of a target product, whereby qualities of the target product are modified.

The modifier and the composition for modification according to the present invention may each be composed only of the starch decomposition product described above as long as the starch decomposition product used in the present technology is contained as an active ingredient. Alternatively, as long as the advantages of the present invention are not impaired, one or more kinds of other components may also be freely selected and contained. As other components, for example, components usually used for formulation, such as excipients, pH adjusters, colorants, flavoring substances, disintegrants, lubricants, stabilizers, and emulsifiers, can be used. Further, components with known or later discovered functions can also be appropriately used together according to the purpose. The starch decomposition product is classified as a food. Therefore, depending on the selection of components other than the starch decomposition product, the modifier and the composition for modification according to the present invention can also be treated as foods.

The method for applying the modifier and the composition for modification according to the present technology to each product is not particularly limited. For example, a method in which the modifier and the composition for modification according to the present technology are blended as they are with each product, and then the starch decomposition product is partially or completely crystallized together with raw materials of the target product, a method in which the modifier according to the present technology in the state of being dissolved or dispersed in an arbitrary solvent is added to each product, and then the starch decomposition product is partially or completely crystallized together with raw materials of the target product, and the like can be mentioned.

### <About Modification Method Using Starch Decomposition Product>

The starch decomposition product used in the present technology is, as described above, crystallized together with raw materials of a target product such as a food or beverage, a pharmaceutical product, a cosmetic product, an industrial product, a feed, a medium, or a fertilizer, whereby qualities of these target products can be modified. That is, the modification method according to the present technology is a method that includes a crystallization step of partially or completely crystallizing the starch decomposition product together with raw materials of a target product.

The method for crystallization in the crystallization step is not particularly limited, and one or more kinds of known crystallization methods can be freely selected and used. In the present technology, for example, a mixture, mixed solution, or dispersion liquid of the starch decomposition product and raw materials of a target product is held, steamed, smoked, or cooled, for example, under predetermined conditions, whereby crystallization with the starch decomposition product can be achieved.

### <About Food or Beverage>

The starch decomposition product used in the present technology can be crystallized together with raw materials of a food or beverage to modify qualities of the food or beverage. Specifically, qualities of a food or beverage, such as humidity resistance, solidifying properties, gelling properties, shape retainability, plasticity, whiteness, water separation resistance, oil separation resistance, mouthfeel, tactile sensation, aftertaste, creaming properties, heat resistance, thickening properties, and aging resistance, can be modified.

The food or beverage for which the present technology can be used is not particularly limited. For example, beverages such as juices, sports drinks, tea, coffee, and black tea, seasonings such as soy sauce and sauces, soups, creams, various dairy products, frozen desserts such as ice creams, various powdered foods (including beverages), foods for preservation, frozen foods, breads, confectioneries, rice, noodles, fishery paste products, meat products, other processed foods, and the like can be mentioned. In addition, the present technology can also be used for foods and beverages with health claims (including food for specified health use, food with function claims, and food with nutrient function claims), so-called health foods (including beverages), liquid foods, baby/infant foods, diet foods, foods for diabetes, and the like.

### <About Pharmaceutical Product>

The starch decomposition product used in the present technology can be crystallized together with raw materials of a pharmaceutical product to modify qualities of the pharmaceutical product. Specifically, qualities of a pharmaceutical product, such as humidity resistance, solidifying properties, gelling properties, shape retainability, plasticity, whiteness, water separation resistance, oil separation resistance, mouthfeel, tactile sensation, aftertaste, creaming properties, heat resistance, thickening properties, and aging resistance, can be modified.

The present technology can be applied to pharmaceutical products aimed at, for example, powdered substrates for powders, granules, and the like, excipients for tablets and the like, suspending agents for liquid preparations, semi-solid preparations, ointment preparations, and the like, osmotic pressure adjusters, coloring agents, carbohydrate sources (calorie sources) for enteral nutrients and the like, etc.

### <About Cosmetic Product>

The starch decomposition product used in the present technology can be crystallized together with raw materials of a cosmetic product to modify qualities of the cosmetic product. Specifically, qualities of a cosmetic product, such as humidity resistance, solidifying properties, gelling properties, shape retainability, plasticity, whiteness, water separation resistance, oil separation resistance, tactile sensation, creaming properties, heat resistance, thickening properties, and aging resistance, can be modified.

The present technology can be applied to cosmetic products aimed at, for example, powdered substrates and excipients for powdery cosmetic products, solid cosmetic products, and the like, suspending agents for liquid, milky, gel, creamy, and like cosmetic products, osmotic pressure adjusters, coloring agents, and the like.

### <About Industrial Product>

The starch decomposition product used in the present technology can be crystallized together with raw materials of an industrial product to modify qualities of the industrial product. Specifically, qualities of an industrial product, such as humidity resistance, solidifying properties, gelling properties, shape retainability, plasticity, whiteness, water separation resistance, oil separation resistance, tactile sensation, creaming properties, heat resistance, thickening properties, and aging resistance, can be modified.

As industrial products to which the present technology can be applied, for example, carriers, various films, fibers, capsules, adhesives, release agents, anti-adhesion agents, bulking agents, abrasives, excipients, and the like can be mentioned.

### <About Feed, Medium, Fertilizer>

The starch decomposition product used in the present technology can be crystallized together with raw materials of a feed, medium, or fertilizer to modify qualities of the feed, medium, or fertilizer. Specifically, qualities of a feed, medium, or fertilizer, such as humidity resistance, solidifying properties, gelling properties, shape retainability, plasticity, whiteness, water separation resistance, oil separation resistance, mouthfeel, tactile sensation, aftertaste, creaming properties, heat resistance, thickening properties, and aging resistance, can be modified.

### <About Method for Producing Food or Beverage, Pharmaceutical Product, Cosmetic Product, Industrial Product, Feed, Medium, or Fertilizer>

The crystallization step in the modification method according to the present technology described above can be performed as a step of a method for producing a food or beverage, a pharmaceutical product, a cosmetic product, an industrial product, a feed, a medium, or a fertilizer to produce a modified food or beverage, pharmaceutical product, cosmetic product, industrial product, feed, medium, or fertilizer.

The timing of performing the crystallization step in the method for producing each product can be freely set according to the production process of each product as long as the advantages of the present invention are not impaired. For example, a method in which the starch decomposition product is added to each semi-finished product at an arbitrary timing in the course of producing each product, and then the starch decomposition product is crystallized together with raw materials of each product, a method in which the starch decomposition product is blended with raw materials of each product, and then the starch decomposition product is crystallized together with each product at an arbitrary timing in the production process of each product, and the like can be mentioned.

### Examples

Hereinafter, the present technology will be described in further detail based on the examples. Incidentally, the examples described below show some typical examples of the present technology, and do not narrow the interpretation of the scope of the present technology.

### <Experiment Example 1>

In Experiment Example 1, how the specific sugar composition of a starch decomposition product affected the modification of a target product was examined. Specifically, the sugar composition and the iodine coloration value of the starch decomposition product used in the present technology were measured.

### (1) Test Method

### [Branching Enzyme]

In this experiment example, as examples of branching enzymes, a potato-derived enzyme purified in according with the method of Eur. J. Biochem., 59, pp. 615 to 625 (1975) (hereinafter referred to as "potato-derived branching enzyme") and Branchzyme (manufactured by Novozymes, hereinafter referred to as "bacteria-derived branching enzyme") were used.

Incidentally, the activity of a branching enzyme was measured by the following method.

As a substrate solution, an amylose solution prepared by dissolving 0.1 mass% of amylose (manufactured by Sigma-Aldrich, A0512) in a 0.1 M acetate buffer (pH 5.2) was used. First, 50 µL of an enzyme solution was added to 50 µL of the substrate solution and allowed to react at 30°C for 30 minutes, and then 2 mL of an iodine-potassium iodide solution (0.39 mM iodine-6 mM potassium iodide-3.8 mM hydrochloric acid mixed solution) was added to stop the reaction. A blank solution was prepared by adding water instead of the enzyme solution. The absorbance at 660 nm was measured 15 minutes after the reaction was stopped. One unit of the amount of enzyme activity of a branching enzyme was defined as the amount of enzyme activity that reduces the absorbance at 660 nm by 1% per minute as tested under the above conditions.

### [Contents with DP 8 to 19 and DP 20 or More]

High performance liquid chromatography (HPLC) analysis was performed under the conditions shown in Table 1 below, and, based on the detected peak area ratio, the contents with a DP of 8 to 19 and a DP of 20 or more were measured.

**[Table 1]**

| Column | MCI CK02AS (manufactured by Mitsubishi Chemical Corporation) |
|---|---|
| Column Temperature | 80°C |
| Eluant | Water |
| Flow Rate | 1.0 mL/min |
| Detector | Differential refractometer |

### [Iodine Coloration Value Measurement]

In a test tube having dispensed therein 5 ml of water, 25 mg (as solids) of a sample (starch decomposition product) was added and mixed. Then, 100 µl of an iodine coloring solution (0.2 mass/vol% iodine and 2 mass/vol% potassium iodide) was added thereto and stirred. After the mixture was allowed to stand at 30°C for 20 minutes, the absorbance at 660 nm was measured with a spectrophotometer using a glass cell having an optical path length of 10 mm, and the difference from the absorbance measurement value in the case of adding no sample was defined as the iodine coloration value.

### (2) Production Method in Examples/Comparative Examples

### [Example 1]

First, 0.2 mass% per solids (g) of α-amylase (Lyquozyme Supra, manufactured by Novozymes Japan Ltd.) was added to 30 mass% of a cornstarch slurry adjusted to pH 5.8 with 10% calcium hydroxide, and liquefied in a jet cooker (temperature: 110°C). The liquefied solution was kept at 95°C, and the DE was measured over time. At the time when the DE became 8, the pH was adjusted to 4 with 10% hydrochloric acid, and the reaction was stopped by boiling. The sugar solution whose reaction had been stopped was adjusted to pH 5.8, and then 1,000 units per solids (g) of a bacteria-derived branching enzyme was added and allowed to react at 50°C for 24 hours. Subsequently, 1.5 mass% per solids (g) of a debranching enzyme (GODO-FIA, manufactured by Godo Shusei Co., Ltd.) was added and allowed to react at 50°C for 24 hours. This starch decomposition product solution was subjected to activated carbon decolorization and ion-exchange purification, and concentrated to a solids concentration of 40 mass%. The concentrate was powdered with a spray dryer, thereby giving a starch decomposition product of Example 1.

### [Example 2]

First, 0.2 mass% per solids (g) of α-amylase (KLEISTASE T10S, manufactured by Amano Enzyme, Inc.) was added to 30 mass% of a cornstarch slurry adjusted to pH 5.8 with 10% calcium hydroxide, and liquefied in a jet cooker (temperature: 110°C). The liquefied solution was kept at 95°C, and the DE was measured over time. At the time when the DE became 9, the pH was adjusted to 4 with 10% hydrochloric acid, and the reaction was stopped by boiling. The sugar solution whose reaction had been stopped was adjusted to pH 5.8, and then 800 units per solids (g) of a bacteria-derived branching enzyme was added and allowed to react at 65°C for 30 hours. Subsequently, 1.0 mass% per solids (g) of a debranching enzyme (GODO-FIA, manufactured by Godo Shusei Co., Ltd.) was added and allowed to react at 50°C for 30 hours. This starch decomposition product solution was subjected to activated carbon decolorization and ion-exchange purification, and concentrated to a solids concentration of 50 mass%. The concentrate was powdered with a spray dryer, thereby giving a starch decomposition product of Example 2.

### [Example 3]

First, 0.2 mass% per solids (g) of α-amylase (KLEISTASE T10S, manufactured by Amano Enzyme, Inc.) was added to 30 mass% of a cornstarch slurry adjusted to pH 5.8 with 10% calcium hydroxide, and liquefied in a jet cooker (temperature: 110°C). The liquefied solution was kept at 95°C, and the DE was measured over time. At the time when the DE became 8, the pH was adjusted to 4 with 10% hydrochloric acid, and the reaction was stopped by boiling. The sugar solution whose reaction had been stopped was adjusted to pH 5.8, and then 600 units per solids (g) of a bacteria-derived branching enzyme was added and allowed to react at 65°C for 15 hours. Subsequently, 0.5 mass% per solids (g) of a debranching enzyme (GODO-FIA, manufactured by Godo Shusei Co., Ltd.) was added and allowed to react at 50°C for 40 hours. This starch decomposition product solution was subjected to activated carbon decolorization and ion-exchange purification, and concentrated to a solids concentration of 45 mass%. The concentrate was powdered with a spray dryer, thereby giving a starch decomposition product of Example 3.

### [Example 4]

First, 30 mass% of a cornstarch slurry adjusted to pH 2 with 10% hydrochloric acid was decomposed to a DE of 13 under a temperature condition of 130°C. After returning to normal pressure, neutralization was performed with 10% sodium hydroxide, the sugar solution whose reaction had thus been stopped was adjusted to pH 5.8, and then 400 units per solids (g) of a bacteria-derived branching enzyme was added and allowed to react at 65°C for 48 hours. Subsequently, 1.0 mass% per solids (g) of a debranching enzyme (GODO-FIA, manufactured by Godo Shusei Co., Ltd.) was added and allowed to react at 50°C for 60 hours. This starch decomposition product solution was subjected to activated carbon decolorization and ion-exchange purification, and powdered with a spray dryer, thereby giving a starch decomposition product of Example 4.

### [Example 5]

First, 0.2 mass% per solids (g) of α-amylase (KLEISTASE T10S, manufactured by Amano Enzyme, Inc.) was added to 30 mass% of a tapioca powder slurry adjusted to pH 5.8 with 10% calcium hydroxide, and liquefied in a jet cooker (temperature: 110°C). The liquefied solution was kept at 95°C, and the DE was measured over time. At the time when the DE became 15, the pH was adjusted to 4 with 10% hydrochloric acid, and the reaction was stopped by boiling. The sugar solution whose reaction had been stopped was adjusted to pH 5.8, and then 2,000 units per solids (g) of a potato-derived branching enzyme was added and allowed to react at 35°C for 30 hours. Subsequently, the pH was adjusted to 4.2, and 1.0 mass% per solids (g) of a debranching enzyme (isoamylase, manufactured by Sigma-Aldrich Japan K.K.) was added and allowed to react at 45°C for 30 hours. This starch decomposition product solution was subjected to activated carbon decolorization and ion-exchange purification, and concentrated to a solids concentration of 60 mass%. The concentrate was powdered with a spray dryer, thereby giving a starch decomposition product of Example 5.

### [Example 6]

First, 30 mass% of a waxy cornstarch slurry adjusted to pH 2 with 10% hydrochloric acid was decomposed to a DE of 6 under a temperature condition of 130°C. After returning to normal pressure, neutralization was performed with 10% sodium hydroxide, the sugar solution whose reaction had thus been stopped was adjusted to pH 5.8, and then 500 units per solids (g) of a bacteria-derived branching enzyme and 0.5 mass% per solids (g) of a debranching enzyme (GODO-FIA, manufactured by Godo Shusei Co., Ltd.) were added and allowed to react at 50°C for 72 hours. This starch decomposition product solution was subjected to activated carbon decolorization and ion-exchange purification, and concentrated to a solids concentration of 40 mass%. The concentrate was powdered with a spray dryer, thereby giving a starch decomposition product of Example 6.

### [Example 7]

First, 0.2 mass% per solids (g) of α-amylase (Lyquozyme Supra, manufactured by Novozymes Japan Ltd.) was added to 30 mass% of a cornstarch slurry adjusted to pH 5.8 with 10% calcium hydroxide, and liquefied in a jet cooker (temperature: 110°C). The liquefied solution was kept at 95°C, and the DE was measured over time. At the time when the DE became 16, the pH was adjusted to 4 with 10% hydrochloric acid, and the reaction was stopped by boiling. The sugar solution whose reaction had been stopped was adjusted to pH 5.8, and then 1,500 units per solids (g) of a bacteria-derived branching enzyme was added and allowed to react at 65°C for 24 hours, followed by boiling to stop the reaction. Subsequently, 1.5 mass% per solids (g) of a debranching enzyme (GODO-FIA, manufactured by Godo Shusei Co., Ltd.) was added and allowed to react at 50°C for 24 hours. This starch decomposition product solution was subjected to activated carbon decolorization and ion-exchange purification, and concentrated to a solids concentration of 55 mass%. The concentrate was powdered with a spray dryer, thereby giving a starch decomposition product of Example 7.

### [Example 8]

First, 20 mass% of a cornstarch slurry adjusted to pH 2 with 10% hydrochloric acid was decomposed to a DE of 15 under a temperature condition of 130°C. After returning to normal pressure, neutralization was performed with 10% sodium hydroxide, the sugar solution whose reaction had thus been stopped was adjusted to pH 5.8, and then 1.0 mass% per solids (g) of a debranching enzyme (isoamylase, manufactured by Sigma-Aldrich Japan KK) was added and allowed to react at 45°C for 50 hours. This starch decomposition product solution was subjected to activated carbon decolorization and ion-exchange purification, and concentrated to a solids concentration of 45 mass%. The concentrate was powdered with a spray dryer, thereby giving a starch decomposition product of Comparative Example 3.

### [Comparative Example 1]

First, 0.2 mass% per solids (g) of α-amylase (Lyquozyme Supra, manufactured by Novozymes Japan Ltd.) was added to 30 mass% of a cornstarch slurry adjusted to pH 5.8 with 10 mass% calcium hydroxide, and liquefied in a jet cooker (temperature: 110°C). The liquefied solution was kept at 95°C, and the DE was measured over time. At the time when the DE became 13, the pH was adjusted to 4 with 10% hydrochloric acid, and the reaction was stopped by boiling. This starch decomposition product solution was subjected to activated carbon decolorization and ion-exchange purification, and concentrated to a solids concentration of 40 mass%. The concentrate was powdered with a spray dryer, thereby giving a starch decomposition product of Comparative Example 1.

### [Comparative Example 2]

First, 0.2 mass% per solids (g) of α-amylase (Lyquozyme Supra, manufactured by Novozymes Japan Ltd.) was added to 30 mass% of a cornstarch slurry adjusted to pH 5.8 with 10 mass% calcium hydroxide, and liquefied in a jet cooker (temperature: 110°C). The liquefied solution was kept at 95°C, and the DE was measured over time. At the time when the DE became 17, the pH was adjusted to 4 with 10% hydrochloric acid, and the reaction was stopped by boiling. This starch decomposition product solution was subjected to activated carbon decolorization and ion-exchange purification, and concentrated to a solids concentration of 40 mass%. The concentrate was powdered with a spray dryer, thereby giving a starch decomposition product of Comparative Example 2.

### [Comparative Example 3]

First, 0.3 mass% per solids (g) of α-amylase (Lyquozyme Supra, manufactured by Novozymes Japan Ltd.) was added to 30 mass% of a cornstarch slurry adjusted to pH 5.8 with 10 mass% calcium hydroxide, and liquefied in a jet cooker (temperature: 110°C). The liquefied solution was kept at 95°C, and the DE was measured over time. At the time when the DE became 30, the pH was adjusted to 4 with 10% hydrochloric acid, and the reaction was stopped by boiling. This starch decomposition product solution was subjected to activated carbon decolorization and ion-exchange purification, and concentrated to a solids concentration of 40 mass%. The concentrate was powdered with a spray dryer, thereby giving a starch decomposition product of Comparative Example 3.

### [Comparative Example 4]

First, 0.2 mass% per solids (g) of α-amylase (Lyquozyme Supra, manufactured by Novozymes Japan Ltd.) was added to 30 mass% of a cornstarch slurry adjusted to pH 5.8 with 10 mass% calcium hydroxide, and liquefied in a jet cooker (temperature: 110°C). The liquefied solution was kept at 95°C, and the DE was measured over time. At the time when the DE became 10, the pH was adjusted to 4 with 10% hydrochloric acid, and the reaction was stopped by boiling. The sugar solution whose reaction had been stopped was adjusted to pH 5.8, and then 0.3 mass% per solids (g) of a debranching enzyme (GODO-FIA, manufactured by Godo Shusei Co., Ltd.) was added and allowed to react at 50°C for 24 hours. Subsequently, 0.2 mass% per solids (g) of α-amylase (KLEISTASE T10S, manufactured by Amano Enzyme, Inc.) was added and heated to 80°C, and then the DE was measured over time. At the time when the DE became 18, the pH was adjusted to 4 with 10% hydrochloric acid, and the reaction was stopped by boiling. This starch decomposition product solution was subjected to activated carbon decolorization and ion-exchange purification, and concentrated to a solids concentration of 40 mass%. The concentrate was powdered with a spray dryer, thereby giving a starch decomposition product of Comparative Example 4.

### [Comparative Example 5]

First, 0.2 mass% per solids (g) of α-amylase (Lyquozyme Supra, manufactured by Novozymes Japan Ltd.) was added to 10 mass% of a waxy cornstarch slurry adjusted to pH 5.8 with 10 mass% calcium hydroxide, and liquefied in a jet cooker (temperature: 110°C). The liquefied solution was kept at 95°C, and the DE was measured over time. At the time when the DE became 6, the pH was adjusted to 4 with 10% hydrochloric acid, and the reaction was stopped by boiling. The sugar solution whose reaction had been stopped was adjusted to pH 5.8, and then 2.0 mass% per solids (g) of a debranching enzyme (GODO-FIA, manufactured by Godo Shusei Co., Ltd.) was added and allowed to react at 50°C for 24 hours. This starch decomposition product solution was subjected to activated carbon decolorization and ion-exchange purification, and powdered with a spray dryer, thereby giving a starch decomposition product of Comparative Example 5.

### (3) Measurement

With respect to the starch decomposition products of Examples 1 to 8 and Comparative Examples 1 to 5 obtained above, the content with a DP of 8 to 19, the content with a DP of 20 or more, and the iodine coloration value were measured by the methods described above. The results are shown in Table 2 below.

**[Table 2]**

| | Examples | | | | | | | | Comparative Examples | | | | |
|---|---|---|---|---|---|---|---|---|---|---|---|---|---|
| | 1 | 2 | 3 | 4 | 5 | 6 | 7 | 8 | 1 | 2 | 3 | 4 | 5 |
| Content with DP 8 to 19 (%) | 53 | 55 | 49 | 50 | 45 | 52 | 36 | 45 | 24 | 16 | 11 | 32 | 42 |
| Content with DP 20 or More (%) | 21 | 24 | 29 | 16 | 11 | 24 | 28 | 28 | 49 | 44 | 21 | 29 | 48 |
| Iodine Coloration Value | 0.75 | 0.64 | 1.28 | 0.35 | 0.15 | 1.05 | 0.22 | 0.72 | 0.35 | 0.15 | 0.01 | 0.11 | 2.48 |

### <Experiment Example 2>

In Experiment Example 2, of the modification effects of the starch decomposition product used in the present technology, examination was performed with respect to solidifying properties and humidity resistance. Specifically, the starch decomposition product used in the present technology was tested for solidifying properties and humidity resistance when used as a binder for a granola bar.

### (1) Production of Granola Bar

Ingredients were mixed and kneaded according to the blending shown in Table 3 below. Each mixture was entwined with 3 g of granola, placed in a mold, and allowed to stand at room temperature for 8 hours to produce a granola bar.

### (2) Evaluation

The solidifying properties and humidity resistance were discussed and evaluated by five special panelists based on the following evaluation criteria.

### [Solidifying Properties]

⊙: Solidified; extremely favorable
○: Slightly solidified; favorable
×: Not solidified; unfavorable

### [Humidity resistance]

Humidity resistance after storage at 25°C and a relative humidity of 90% for 15 hours
⊙: No moisture absorption, no stickiness; extremely favorable
○: Slight moisture absorption, stickiness felt; yet favorable
×: Deliquesced; unfavorable

### (3) Results

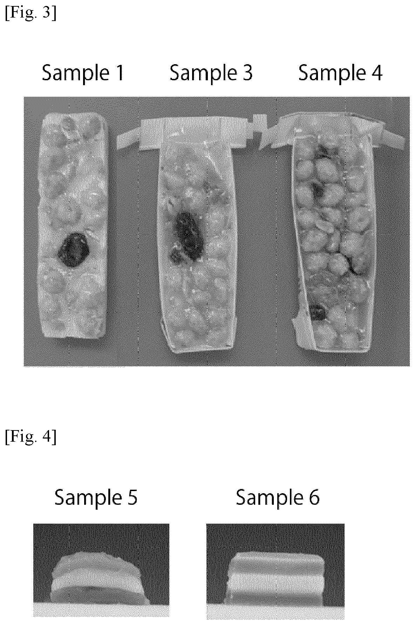

The results are shown in Table 3. In addition, photographs of Samples 1, 3, and 4 are shown in Fig. 3. Samples 3 and 4 did not solidify and were semi-solid or liquid.

**[Table 3]**

| | | Samples | | | |
|---|---|---|---|---|---|
| | | 1 | 2 | 3 | 4 |
| Blending (part by mass) | Starch Decomposition Product of Example 1 | 8.0 | 8.0 | - | - |
| | Starch Decomposition Product of Comparative Example 2 | - | - | 8.0 | - |
| | Lactosucrose (Moisture Content: 75%)¹⁾ | 4.0 | - | 4.0 | 15.0 |
| | Fructo Oligosaccharide (Moisture Content: 75%)²⁾ | - | 4.0 | - | - |
| | Water | 4.0 | 3.0 | 3.0 | - |
| Evaluation | Solidifying Properties | ⊙ | ⊙ | × | × |
| | Moisture Absorption Resistance | ⊙ | ⊙ | - | - |

| | | | | | |
|---|---|---|---|---|---|
| 1) Oligo-no-Okage (Ensuiko Sugar Refining Co., Ltd.) 2) Meioligo G (Meiji Food Materia Co., Ltd.) | | | | | |

As shown in Table 3, it turned out that Samples 1 and 2 prepared using the starch decomposition product of Example 1 solidify oligosaccharides, which normally do not solidify, to bind granola, and, despite the presence of oligosaccharides that easily absorb moisture, humidity resistance is imparted. Meanwhile, in Sample 3 prepared using the starch decomposition product of Comparative Example 2, solidification was not possible (see Fig. 3).

### <Experiment Example 3>

In Experiment Example 3, of the modification effects of the starch decomposition product used in the present technology, examination was performed with respect to shape retainability, whiteness, and tactile sensation improvability. Specifically, the starch decomposition product used in the present technology was tested for shape retainability, whiteness, tactile sensation improvability (stickiness), and mouthfeel (ease of biting) when applied to *uiro* (sweet rice jelly).

### (1) Production of Uiro

First, 20 parts by mass of water was added to the ingredients shown in Table 4 below, and thoroughly stirred. The mixture was placed in a mold and steamed for 20 minutes, then stored at 4°C for 1 hour, and removed from the mold to produce *uiro.*

### (2) Evaluation

The shape retainability, whiteness, tactile sensation improvability (stickiness), and mouthfeel (ease of biting) were discussed and evaluated by five special panelists based on the following evaluation criteria.

### [Shape Retainability]

⊙: Hard, with shape retainability maintained
○: Slightly hard, with shape retainability present
×: Soft, with no shape retainability

### [Whiteness]

⊙: White
○: Slightly white
×: Not white

### [Tactile Sensation Improvability (Stickiness)]

⊙: Not sticky; extremely favorable
○: Slightly sticky; yet favorable
×: Sticky; unfavorable

### [Mouthfeel (Ease of Biting)]

⊙: Easy to bite; extremely favorable
○: Slightly easy to bite; favorable
×: Hard to bite; unfavorable

### (3) Results

The results are shown in Table 4. In addition, photographs of Samples 5 and 6 are shown in Fig. 4.

**[Table 4]**

| | | Samples | | | |
|---|---|---|---|---|---|
| | | 5 | 6 | 7 | 8 |
| Blending (part by mass) | Rice Flour | 10.0 | 10.0 | 10.0 | - |
| | Flour | - | - | - | 10.0 |
| | Sugar | 10.0 | 8.0 | 8.0 | 10.0 |
| | Starch Decomposition Product of Example 3 | - | 2.0 | - | - |
| | Starch Decomposition Product of Comparative Example 2 | - | - | 2.0 | - |
| Results | Shape Retainability | × | ○ | × | ⊙ |
| | Whiteness | ○ | ⊙ | ○ | × |
| | Tactile Sensation Improvability (Stickiness) | × | ○ | ○ | ⊙ |
| | Mouthfeel (Ease of Biting) | × | ⊙ | ○ | × |

As shown in Table 4 and Fig. 4, it turned out that when the starch decomposition product of Example 3 is used for *uiro,* shape retainability that prevents collapse even when cut and whiteness that allows a colorant to stand out are imparted, and mouthfeel with good elasticity and ease of biting is obtained.

### <Experiment Example 4>

In Experiment Example 4, of the modification effects of the starch decomposition product used in the present technology, examination was performed with respect to shape retainability and water separation resistance. Specifically, the starch decomposition product used in the present technology was tested for shape retainability and water separation resistance when applied to a whipped cream.

### (1) Production of Whipped Cream

Using a bowl cooled with ice water, 200 g of fresh cream and 20 g of each of the ingredients shown in Table 5 below were mixed, and then fully whipped using an electrical mixer to produce a whipped cream. Each whipped cream produced was shaped using a decorating tube.

### (2) Evaluation

The shape retainability and water separation resistance were discussed and evaluated by five special panelists based on the following evaluation criteria.

### [Shape Retainability]

Shape retainability after storing the shaped whipped cream at 4°C for 24 hours
⊙: Has shape retainability; extremely favorable
Δ: Slightly lacks shape retainability; slightly unfavorable
×: Has no shape retainability; unfavorable

### [Water Separation Resistance]

Water separation resistance after storing the shaped whipped cream at 4°C for 24 hours
⊙: No water separation; extremely favorable
Δ: Local water separation is seen; slightly unfavorable
×: Water separation is seen; unfavorable

### (3) Results

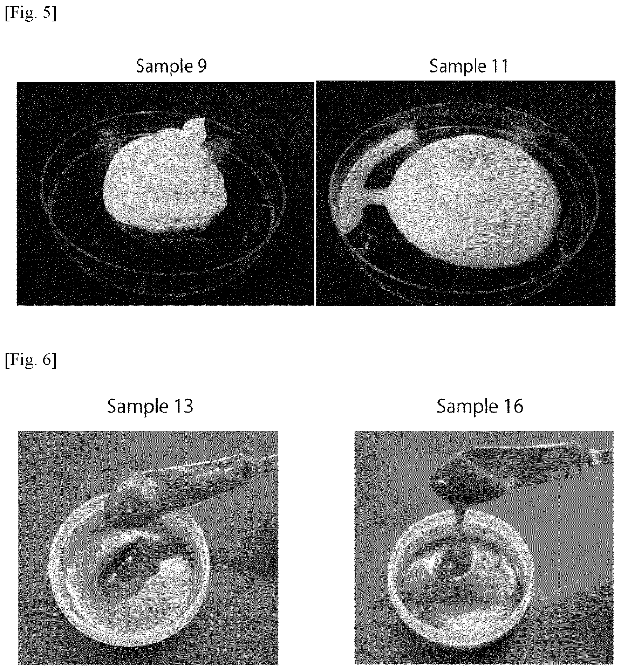

The results are shown in Table 5. In addition, photographs of Samples 9 and 11 after being stored at 4°C for 24 hours are shown in Fig. 5.

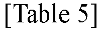

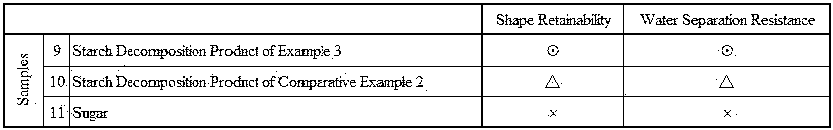

As shown in Table 5 and Fig. 5, it turned out that when the starch decomposition product of Example 3 is used for a whipped cream, shape retainability that prevents collapse during storage and water separation resistance are imparted.

### <Experiment Example 5>

In Experiment Example 5, of the modification effects of the starch decomposition product used in the present technology, examination was performed with respect to creaming properties and shape retainability. Specifically, the starch decomposition product used in the present technology was examined for plasticity and creaming properties when applied to a fat spread.

### (1) Production of Fat Spread

Eight parts by mass of each of the starch decomposition products of Examples 2 to 5 (Samples 12 to 15) and the starch decomposition product of Comparative Example 2 (Sample 16), 25 parts by mass of canola oil, 4 parts by mass of sugar, 1 part by mass of cocoa powder, 2 parts by mass of skim milk powder, and 10 parts by mass of water were weighed, dissolved and stirred in a stirrer, transferred to a container, and stored at 4°C for 24 hours to produce a fat spread.

### (2) Evaluation

The plasticity was discussed and evaluated by five special panelists based on the following evaluation criteria.

### [Plasticity]

⊙: Plastic; extremely favorable
○: Slightly plastic; favorable
×: Not plastic; unfavorable

### (3) Results

The evaluation results of plasticity are shown in Table 6. Photographs of the fat spreads of Sample 13 and Sample 16 produced are shown in Fig. 6.

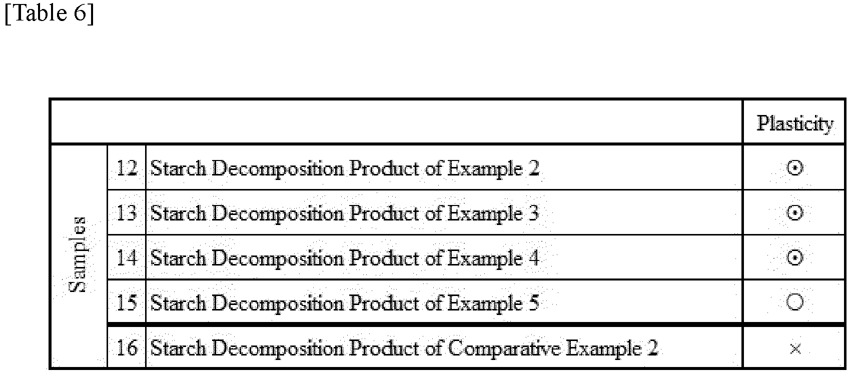

As shown in Table 6, Samples 12 to 15 prepared using the starch decomposition products of Examples 2 to 5 had plasticity, but Sample 16 prepared using the starch decomposition product of Comparative Example 2 did not have plasticity. From the comparison of Samples 12 to 15 prepared using the starch decomposition products of Examples 2 to 5, it turned out that a higher iodine coloration value leads to better plasticity.

In addition, as shown in Fig. 6, Sample 13 prepared using the starch decomposition product of Example 3 solidified like a cream, had shape retainability, was smooth and easy to spread on bread, and thus was a fat spread with favorable plasticity. Meanwhile, Sample 16 prepared using the starch decomposition product of Comparative Example 2 did not solidify and was liquid.

From these results, it turned out that use of the present technology makes it possible to form any liquid oil (e.g., omega-3 fatty acid-containing oil, sesame oil, olive oil, etc.) into a spread, and that a low-calorie spread with excellent usability and mouthfeel can be easily produced without using hydrogenated fats or saturated fatty acid-based oils, whose high intake is considered undesirable,.

### <Experiment Example 6>

In Experiment Example 6, of the modification effects of the starch decomposition product used in the present technology, examination was performed with respect to solidifying properties and heat resistance. Specifically, the starch decomposition product used in the present technology was tested for solidifying properties and heat resistance when applied to a chocolate-like confectionery.

### (1) Production of Chocolate-Like Confectionery

In a bowl, 22 parts by mass of each of the starch decomposition products of Examples 1, 4, 7, and 8 (Samples 17 to 20) and the starch decomposition products of Comparative Examples 1 and 4 (Samples 21 and 22), 4 parts by mass of canola oil, 3 parts by mass of sugar, 1 part by mass of cocoa powder, 2 parts by mass of skim milk powder, and 8 parts by mass water were weighed, well dissolved using a rubber spatula, transferred to a mold, and stored at room temperature for 24 hours to produce a chocolate-like confectionery.

### (2) Evaluation

The hardness (solidifying properties) was discussed and evaluated by five special panelists based on the following evaluation criteria.

### [Hardness (Solidifying Properties)]

⊙: Hard; extremely favorable
○: Slightly hard; favorable
×: Liquid; unfavorable

### (3) Results

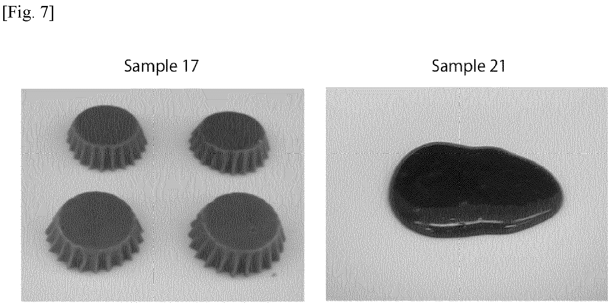

The evaluation results of hardness (solidifying properties) are shown in Table 7 below. In addition, photographs of the chocolate-like confectioneries of Samples 17 and 21 produced are shown in Fig. 7.

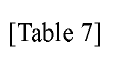

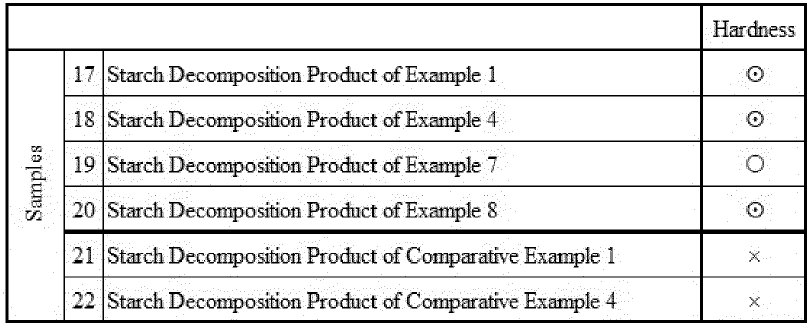

As shown in Table 7 and Fig. 7, Samples 17 to 20 prepared using the starch decomposition products of Examples 1, 4, 7, and 8 became solid, resulting in confectioneries close to chocolates in hardness and mouthfeel. In addition, Samples 17 to 20 did not change even when heated in an oven at 250°C for 5 minutes. Meanwhile, Samples 21 and 22 prepared using the starch decomposition products of Comparative Examples 1 and 4 did not solidify and were liquid.

From these results, it turned out that use of the present technology makes it possible to solidify any liquid oil (e.g., omega-3 fatty acid-containing oil, sesame oil, olive oil, etc.), and that low-fat, low-calorie, and highly heat-resistant chocolate-like confectioneries can be easily produced without using cocoa mass or cocoa butter.

### <Experiment Example 7>

In Experiment Example 7, of the modification effects of the starch decomposition product used in the present technology, examination was performed with respect to thickening properties and aging resistance. Specifically, the starch decomposition product used in the present technology was tested for whiteness, sweet taste properties (sweetness), aftertaste, thickening properties (viscosity), oil separation resistance (oil floating), and aging resistance when applied to a sesame dressing.

### (1) Production of Sesame Dressing

Nine parts by mass of each of the starch decomposition products of Example 1 and 6 (Samples 23 and 24) and the starch decomposition products of Comparative Examples 1, 3, and 5 (Samples 25 to 27), 3 parts by mass of sugar, 2 parts by mass of ground sesame, 1 part by mass of vinegar, 1 part by mass of soy sauce, 9 parts by mass of water, and 6 parts by mass of sesame oil were weighed, dissolved and stirred in a stirrer, transferred to a container, and stored at 4°C for 24 hours to produce a sesame dressing.

### (2) Evaluation

The whiteness, sweet taste properties (sweetness), aftertaste, thickening properties (viscosity), oil separation resistance (oil floating), and aging resistance were discussed and evaluated by five special panelists based on the following evaluation criteria.

### [Whiteness]

⊙: White
○: Slightly white
×: Not white

### [Sweet Taste Properties (Sweetness)]

⊙: Extremely favorable
○: Favorable
×: Too sweet

### [Aftertaste]

⊙: Extremely favorable
○: Favorable
×: Unfavorable

### [Thickening Properties (Viscosity)]

⊙: Extremely favorable
○: Favorable
×: Unfavorable

### [Oil Separation Resistance (Oil Floating)]

⊙: No oil floating
○: Slight oil floating
×: Oil floating is seen

### [Aging Resistance]

Aging resistance of the sesame dressing after being stored at 25°C for 1 week
⊙: Viscosity is maintained
×: Viscosity is not maintained

### (3) Results

The evaluation results of whiteness, sweet taste properties (sweetness), aftertaste, thickening properties (viscosity), oil separation resistance (oil floating), and aging resistance are shown in Table 8.

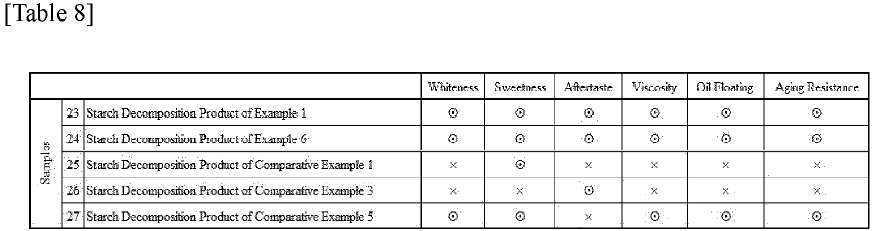

As shown in Table 8, the sesame dressings of Samples 23 and 24 prepared using the starch decomposition products of Examples 1 and 6 were both white with viscosity, had favorable sweetness/aftertaste, and had smooth mouthfeel. In addition, the sesame dressings of Samples 23 and 24 showed no oil floating when shaken well and allowed to stand for 30 minutes. Further, even after storage at 25°C for 1 week, the viscosity was maintained, and the mouthfeel did not change. Meanwhile, the sesame dressings of Samples 25 and 26 prepared using the starch decomposition products of Comparative Examples 1 and 3 were all liquid without viscosity. In addition, after being shaken well and left to stand for 30 minutes, they showed oil floating. Further, Sample 25 had a bad aftertaste, and an unpleasant taste was felt. In addition, Sample 27 prepared using Comparative Example 5 was white and had viscosity, and the viscosity was maintained even after storage at 25°C for 1 week. Further, Sample 27 showed no oil floating after being shaken well and allowed to stand for 30 minutes, but had a bad aftertaste.

From these results, it was turned out that use of the present technology makes it possible to increase the viscosity of a sauce or dressing without using thickening polysaccharides classified as food additives. In addition, it also turned out that use of the present technology does not require heating, which is normally required when using starches, and can also prevent aging during storage. Further, it also turned out that use of the present technology makes it possible to impart oil separation resistance and suppress oil floating.

### <Experiment Example 8>

In Experiment Example 8, of the modification effects of the starch decomposition product used in the present technology, examination was performed with respect to solidifying properties and mouthfeel improvability. Specifically, the starch decomposition product used in the present technology was tested for solidifying properties and mouthfeel improvability when applied to an unbaked cheesecake.

### (1) Production of Unbaked Cheesecake

In a bowl, 18 parts by mass of a starch decomposition product (Example 3 or Comparative Example 1), 30 parts by mass of fresh cream, 4 parts by mass of sugar, and 2 parts by mass of grated cheese were weighed and well dissolved using a rubber spatula. The mixture was transferred to a container and stored at 4°C for 24 hours to produce an unbaked cheesecake of Sample 28 or 29.

In addition, 20 parts by mass of a starch decomposition product (Example 3), 20 parts by mass of milk, 4 parts by mass of salad oil, 4 parts by mass of sugar, and 2 parts by mass of grated cheese were weighed and well dissolved using a rubber spatula. The mixture was transferred to a container and stored at 4°C for 24 hours to produce an unbaked cheesecake of Sample 30.

### (2) Results

The unbaked cheesecakes of Samples 28 and 30 prepared using the starch decomposition product of Example 3 both solidified and had smooth mouthfeel. Meanwhile, the unbaked cheesecake of Sample 29 prepared using the starch decomposition product of Comparative Example 1 remained liquid.

From these results, it turned out that use of the present technology makes it possible to solidify dough without using a solidifying agent such as gelatin, and smooth mouthfeel can be obtained without using cream cheese or the like.

### <Experiment Example 9>

In Experiment Example 9, of the modification effects of the starch decomposition product used in the present technology, examination was performed with respect to creaming properties, mouthfeel improvability, and aging resistance. Specifically, the starch decomposition product used in the present technology was tested for creaming properties, mouthfeel improvability, and aging resistance when applied to a milk filling.

### (1) Production of Milk Filling

In a bowl, 5 parts by mass of a starch decomposition product (Example 1 or Comparative Example 2), 2.5 parts by mass of sugar, 2.5 parts by mass of skim milk powder, 4 parts by mass of salad oil, and 6 parts by mass of water were weighed, and dissolved and stirred in a stirrer. The mixture was transferred to a container and stored at 4°C for 24 hours to produce a milk filling of Sample 31 or 32.

### (2) Results

The milk filling of Sample 31 prepared using the starch decomposition product of Example 1 was creamy and had smooth mouthfeel. Meanwhile, the milk filling of Sample 32 prepared using the starch decomposition product of Comparative Example 2 remained liquid.

From these results, it turned out that use of the present technology makes it possible to cream a filling, and, because heating, which is required for gelatinization in general flour paste production, is not applied, the flavor does not deteriorate, and aging does not occur, allowing for low-temperature storage, which also makes a sanitary contribution.

### <Experiment Example 10>

In Experiment Example 10, of the modification effects of the starch decomposition product used in the present technology, examination was performed with respect to solidifying properties and humidity resistance. Specifically, the starch decomposition product used in the present technology was tested for solidifying properties and humidity resistance when applied to a powdered substrate.

### (1) Production of Powdered Product 1

In a bowl, the ingredients shown in Table 9 below were weighed and kneaded. The mixture was transferred to a container and stored at 60°C for 12 hours to cause crystallization. The resulting solidified product was pulverized in a mill to produce each powdered product of Samples 33 to 36. Incidentally, Sample 36 prepared using the starch decomposition product of Comparative Example 2 did not solidify, and it was not possible to produce a powdered product.

**[Table 9]**

| | | Samples | | | |
|---|---|---|---|---|---|
| | | 33 | 34 | 35 | 36 |
| Blending (part by mass) | Starch Decomposition Product of Example 3 | 12.0 | 10.0 | 10.0 | - |
| | Starch Decomposition Product of Comparative Example 2 | - | - | - | 12.0 |
| | Fructo Oligosaccharide ²⁾ | 4.0 | - | - | 4.0 |
| | Soy Sauce | - | 5.0 | - | - |
| | Salad Oil | - | - | 2.0 | - |
| | Water | 5.0 | - | 4.0 | 5.0 |

### (2) Production of Powdered Product 2

First, 25 g of the starch decomposition product of Example 1, 25 g of water, and 500 µL of a 0.1 N iodine solution were placed in a vial and stirred at room temperature for 3 days using a stirrer to cause precipitation. The precipitate-containing solution was diluted and filtered, and the obtained filtrate was washed with water, air-dried, and then pulverized to produce a powdered product of Sample 37. The powder was purple, confirming the incorporation of iodine.

### (3) Results

Samples 33 to 35 and 37 prepared using the starch decomposition product of Example 1 or 3 were successfully powdered. The powdered products all remained powdery without absorbing moisture even when stored at 25°C and a high relative humidity of 94% for 24 hours.

From these results, it turned out that use of the present technology makes it possible to powder a wide range of products. Specifically, it turned out that a powder having humidity resistance can be obtained by being spray-dried together with the modifier according to the present technology even with substances such as oligosaccharides and soy sauce that easily absorb humidity. In addition, it turned out that even oils and the like, whose drying normally requires spray-drying after an emulsification treatment, can be easily powdered. Further, it also turned out that substances known to be incorporated by amylose, such as iodine and fatty acids, can be incorporated through the inclusion action and powdered.

### <Experiment Example 11>

In Experiment Example 11, of the modification effects of the starch decomposition product used in the present technology, examination was performed with respect to shape retainability, whiteness, and aftertaste. Specifically, the starch decomposition product used in the present technology was tested for shape retainability, whiteness, and aftertaste when applied to an almond jelly.

### (1) Production of Almond Jelly

The ingredients shown in Table 10 below were stirred and dissolved at 65°C, transferred to a mold, and hardened at 4°C to obtain almond jelly.

### (2) Evaluation

The shape retainability, whiteness, and aftertaste were discussed and evaluated by five special panelists based on the following evaluation criteria.

### [Shape Retainability]

⊙: Has shape retainability; extremely favorable
○: Has slight shape retainability; favorable
×: Has no shape retainability; unfavorable

### [Whiteness]

⊙: White
○: Slightly white
×: Not white

### [Aftertaste]

⊙: Extremely favorable
○: Favorable
×: Unfavorable

### (3) Results

The evaluation results of shape retainability, whiteness, and aftertaste are shown in Table 10 below.

**[Table 10]**

| | | Samples | | | |
|---|---|---|---|---|---|
| | | 38 | 39 | 40 | 41 |
| Blending (part by mass) | Starch Decomposition Product of Example 2 | 10.0 | - | - | - |
| | Starch Decomposition Product of Example 7 | - | 10.0 | - | - |
| | Starch Decomposition Product of Comparative Example 2 | - | - | 10.0 | - |
| | Starch Decomposition Product of Comparative Example 5 | - | - | - | 10.0 |
| | Skim Milk Powder | 5.0 | 5.0 | 5.0 | 5.0 |
| | Sugar | 5.0 | 5.0 | 5.0 | 5.0 |
| | Almond Powder | 2.0 | 2.0 | 2.0 | 2.0 |
| | Gelatin | 1.0 | 1.0 | 1.0 | 1.0 |
| | Water | 27.0 | 27.0 | 27.0 | 27.0 |
| Results | Shape Retainability | ⊙ | ○ | × | ⊙ |
| | Whiteness | ⊙ | ⊙ | × | ⊙ |
| | Aftertaste | ⊙ | ⊙ | × | × |

As shown in Table 10, Samples 38 and 39 prepared using the starch decomposition products of Example 2 or Example 7 had excellent shape retainability and whiteness, and an almond jelly with a good aftertaste was obtained. Meanwhile, Sample 40 prepared using the starch decomposition product of Comparative Example 2 was poor in all the evaluations. In addition, in Sample 41 prepared using the starch decomposition product of Comparative Example 5, although the shape retainability and whiteness were excellent, the aftertaste was unfavorable.

From these results, it turned out that a higher iodine coloration value leads to better shape retainability and whiteness, and a lower content with a DP of 20 or more leads to a better aftertaste.

### <Experiment Example 12>

In Experiment Example 12, of the modification effects of the starch decomposition product used in the present technology, examination was performed with respect to solidifying properties. Specifically, the starch decomposition product used in the present technology was tested for solidifying properties when applied to a nursing care food.

### (1) Production/Evaluation of Nursing Care Food

Boiled vegetables (taro, carrot, kidney beans, burdock) and chicken each in an amount of 20 parts by mass, 12 parts by mass of a starch decomposition product (Example 3 or Comparative example 1), 1 part by mass of salad oil, 5 parts by mass of soup stock were added and crushed in a mixer to a uniform mixture. The mixture was transferred to a container and held at 4°C for 4 hours to produce a nursing care food of Sample 42 or 43. As a result, Sample 42 prepared using the starch decomposition product of Example 3 became shapable due to solidification, while Sample 43 prepared using the starch decomposition product of Comparative Example 1 remained liquid.

Sample 42 was reshaped into the shapes of the vegetables and chicken and served on a plate, and a warm seasoning liquid at about 40°C was poured thereon to produce a reshaped food as a simmered dish. As a result, each of the reshaped ingredients was solid and stable, but hardly offered a feeling of fibers when eaten, and was easy to chew.

From these results, it turned out that use of the present technology makes it possible to produce a reshaped food that is easy to chew simply by mixing with ingredients with crushing without heating, and carbohydrates can also be replenished at the same time.

## Claims

1. A modifier comprising a starch decomposition product in which
the content with a glucose polymerization degree (DP) of 8 to 19 is 32% or more, and
the content with a glucose polymerization degree (DP) of 20 or more is 30% or less,
the modifier being configured to partially or completely crystallize the starch decomposition product together with raw materials of a target product, thereby modifying qualities of the target product.

2. The modifier according to claim 1, wherein the starch decomposition product has an iodine coloration value of 0.15 or more.

3. The modifier according to claim 1 or 2, wherein the starch decomposition product is obtained by allowing at least a debranching enzyme and a branching enzyme to act on a starch or a starch decomposition intermediate.

4. The modifier according to claim 1 or 2, wherein the starch decomposition product is obtained by allowing at least a debranching enzyme to act on a starch or a starch decomposition intermediate that has been liquefied with an acid.

5. The modifier according to any one of claims 1 to 4, wherein the target product is a food or beverage, a pharmaceutical product, a cosmetic product, an industrial product, a feed, a medium, or a fertilizer.

6. A composition for modification, comprising the modifier according to any one of claims 1 to 5.

7. A food or beverage, a pharmaceutical product, a cosmetic product, an industrial product, a feed, a medium, or a fertilizer comprising the modifier according to any one of claims 1 to 5 or the composition for modification according to claim 6.

8. A food or beverage, a pharmaceutical product, a cosmetic product, an industrial product, a feed, a medium, or a fertilizer comprising a starch decomposition product in which
the content with a glucose polymerization degree (DP) of 8 to 19 is 32% or more, and
the content with a glucose polymerization degree (DP) of 20 or more is 30% or less, and which has been partially or completely crystallized together with raw materials of the food or beverage, pharmaceutical product, cosmetic product, industrial product, feed, medium, or fertilizer.

9. A method for modifying a target product, comprising a crystallization step of partially or completely crystallizing, together with raw materials of the target product, a starch decomposition product in which
the content with a glucose polymerization degree (DP) of 8 to 19 is 32% or more, and
the content with a glucose polymerization degree (DP) of 20 or more is 30% or less.
